(19) Europäisches Patentamt

European Patent Office

Office européen des brevets

(11) **EP 1 457 184 A1**

(12) **EUROPEAN PATENT APPLICATION**

(43) Date of publication:
**15.09.2004 Bulletin 2004/38**

(51) Int Cl.[7]: **A61F 13/15**, A61L 15/20,
A61L 15/22, A61L 15/28

(21) Application number: **04251198.0**

(22) Date of filing: **02.03.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **14.03.2003 US 390533**

(71) Applicant: **Weyerhaeuser Company
Federal Way, WA 98063-9777 (US)**

(72) Inventor: **West, Hugh
Seattle, WA 98115 (US)**

(74) Representative: **Hayes, Adrian Chetwynd et al
Boult Wade Tennant,
Verulam Gardens
70 Gray's Inn Road
London WC1X 8BT (GB)**

(54) **Saccharide treated cellulose pulp sheets**

(57)     Cellulose pulp sheets treated with a saccharide yield less than 20 wt. % knots when fiberized under standard Kamas conditions. The fiberized sheets when airlaid produce fiber webs that exhibit desirable densification properties and softness properties.

EP 1 457 184 A1

# EP 1 457 184 A1

**Description**

FIELD OF THE INVENTION

The present invention relates to cellulose fibers that have been treated with a saccharide in order to modify the properties of the cellulose fibers and to methods for applying saccharides to cellulose fibers.

BACKGROUND OF THE INVENTION

Cellulose fibers have found widespread application in absorbent articles, such as diapers and feminine hygiene products. The cellulose fibers are generally used as an absorbent medium to acquire, transport, and hold fluids. While cellulose fibers are effective at acquiring, transporting, and holding fluids, many improvements to cellulose fibers have been made over the past decades to improve the performance properties of cellulose fibers in absorbent products. For example, U.S. Patent Nos. 6,340,411 and 5,547,541 describe that webs of cellulose fibers treated with certain polymeric and nonpolymeric materials require less pressure to densify the web to a given density as compared to the pressure needed to densify a similar web without the polymeric or nonpolymeric material present.

The cellulose fibers treated with the compositions described in U.S. Patent No. 5,547,541 are manufactured by applying the desired compositions to a wet laid web of cellulose fibers which has been produced, for example, using a Fourdrinier machine. The treated wet laid web of cellulose fibers is generally formed into a roll for bulk delivery to an absorbent product manufacturer. The absorbent product manufacturer typically unrolls the roll and processes the web in a fiberization unit that individualizes the fibers and prepares them for further processing.

Several challenges are faced by absorbent product manufacturers when producing products from the treated cellulose fibers discussed above. The ability of the fibers to be individualized affects both the visual appearance of the final product and the ability of the resulting fluff to perform its function of separating and distributing the superabsorbent particles which are typically admixed with it. Knots are defined as clumps or bunches of fibers that have not been individualized. It is well known that the utility and acceptability of a mat of fibers decreases with an increasing level of knot content.

The absorbent product industry is a competitive industry where there is constant downward pressure on the cost of raw materials. The search for desirable treatment chemicals is limited by the need of the industry to use chemicals which are safe and which are not susceptible to a negative perception by the consuming public.

U.S. Patent No. 3,903,889 describes a process for adhering absorbent particles to fibers, an airlaid web of crepe paper, or tissue paper using corn syrup solids, honey, and dextrins which can be diluted with organic solvents. U.S. Patent No. 5,789,326 which discusses U.S. Patent No. 3,903,889 notes that corn syrup is not a hygroscopic material and that corn syrup is excluded as an acceptable binder for attaching superabsorbent particles to fibers in some embodiments because corn syrup remains tacky upon drying. U.S. Patent No. 5,789,326 also describes that such tacky binders make processing of binder coated fibers difficult, e.g., the application of neat corn syrup to cellulose fibers makes the fibers more difficult to fiberize. Poorly fiberized cellulose fibers airlaid into webs are less desirable compared to airlaid webs of fibers that are fiberized to a greater extent. Heretofore, corn syrup has not found use in the manufacture of customized cellulose fibers which have been treated to modify their properties, despite the favorable economics of using corn syrup. Manufacturers of absorbent products continue to look for effective and economical alternatives to existing cellulose fibers for use in their products.

SUMMARY OF THE INVENTION

Surprisingly, the present inventors have observed that cellulose pulp sheets treated with a saccharide in accordance with the present invention are readily fiberizable within the requirements of absorbent product manufacturers. Fibers resulting from the fiberization of cellulose pulp sheets treated with saccharides in accordance with the present invention exhibit densification and softness properties that are superior to densification and softness properties of fibers resulting from cellulose pulp sheets that have not been treated in accordance with the present invention. Absorbent product manufacturers will find saccharide treated cellulose pulp sheets desirable because of their fiberization properties and the densification and softness properties of the fibers produced from the treated pulp sheets. In addition, absorbent product manufacturers will consider saccharides desirable because of their low cost and perceived safety by the consuming public and the absence of any negative perception.

In one embodiment, the present invention relates to a cellulose pulp sheet that includes cellulose fibers, and a saccharide, wherein the pulp sheet when fiberized under standard Kamas conditions yields less than 20 wt. % knots.

In another embodiment, the present invention relates to a method of producing a cellulose pulp sheet that when fiberized under standard Kamas conditions yields less than 20 wt. % knots. The method includes providing a cellulose pulp sheet and applying a saccharide to the cellulose pulp sheet.

In a preferred embodiment, the cellulose pulp sheet consists essentially of cellulose fibers, a saccharide, and water introduced with the saccharide as it is applied to the cellulose pulp sheet.

In another embodiment, the present invention is a cellulose pulp in non-sheet form that includes a mass of cellulose fibers, wherein the mass has a density of at least 0.3 g/cm$^3$; and a saccharide, wherein the cellulose pulp in non-sheet form when fiberized under standard Kamas conditions yields less than 20 wt. % knots.

The present invention provides absorbent product manufacturers with a source of cellulose pulp sheets that exhibit desirable fiberization properties resulting in fibers that exhibit desirable densification and softness properties.

BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:

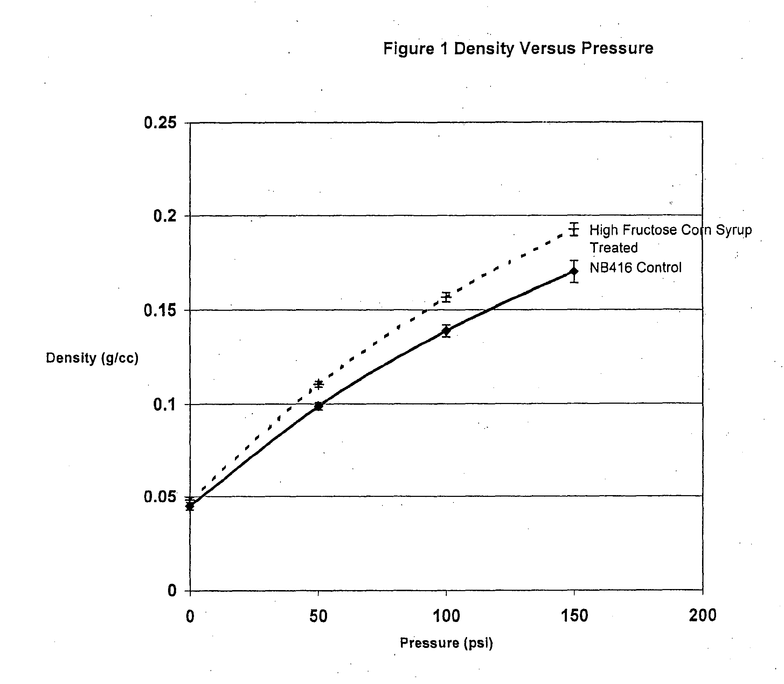

FIGURE 1 is a graph illustrating the difference in densification properties between fibers resulting from the fiberization of high fructose corn syrup treated cellulose pulp sheets of the present invention and fibers resulting from the fiberization of cellulose pulp sheets which have not been treated in accordance with the present invention;

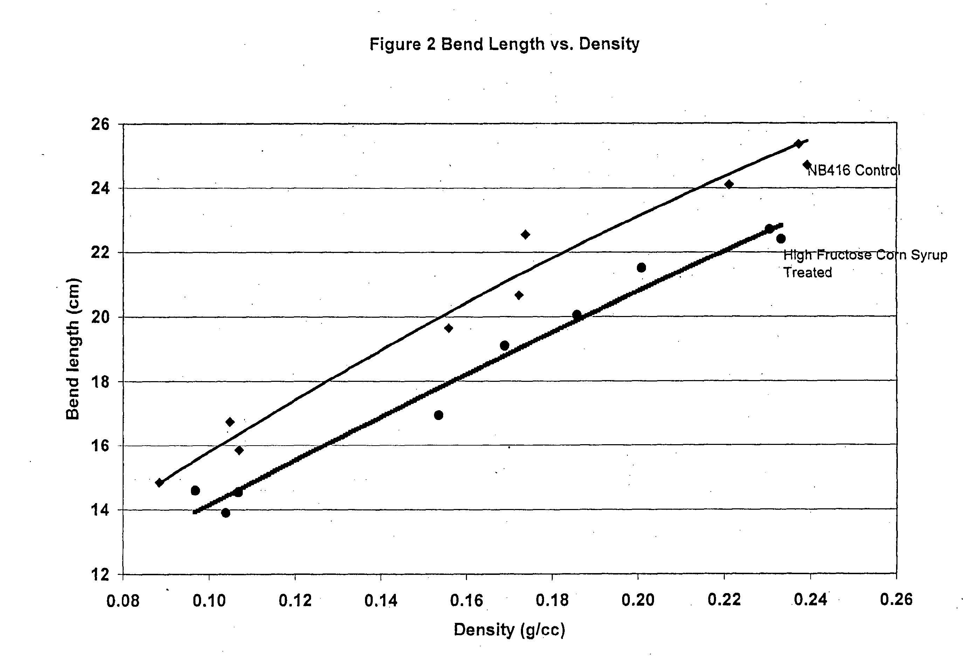

FIGURE 2 is a graph illustrating the softness properties of fibers resulting from the fiberization of high fructose corn syrup treated cellulose pulp sheets of the present invention compared to the softness properties of fibers resulting from the fiberization of cellulose pulp sheets that have not been treated in accordance with the present invention;

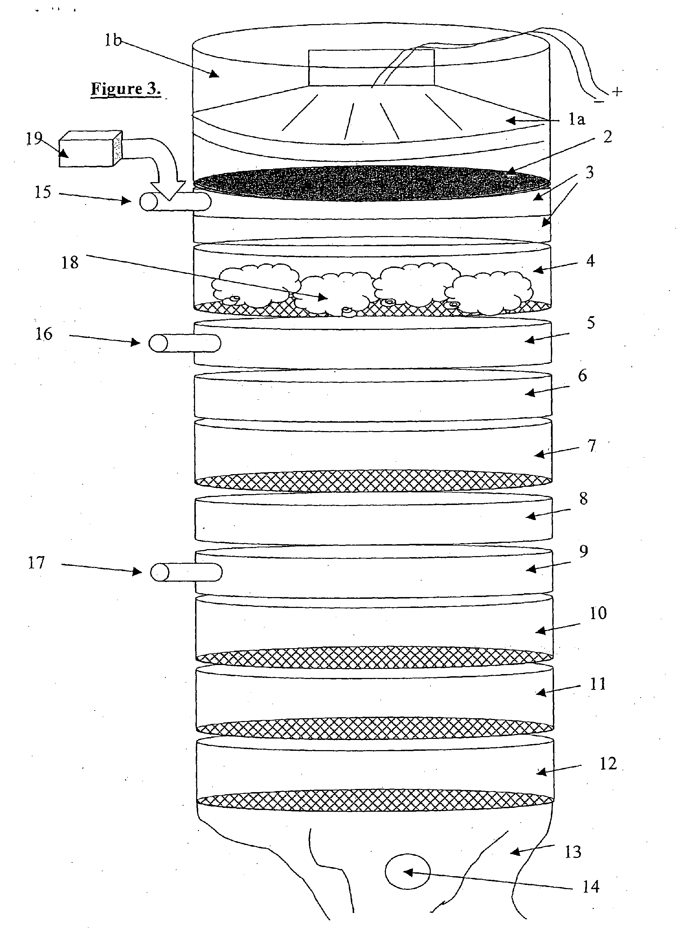

FIGURE 3 is a schematic illustration of a sonic fractionator used to determine the knot level of fiberized cellulose fibers;

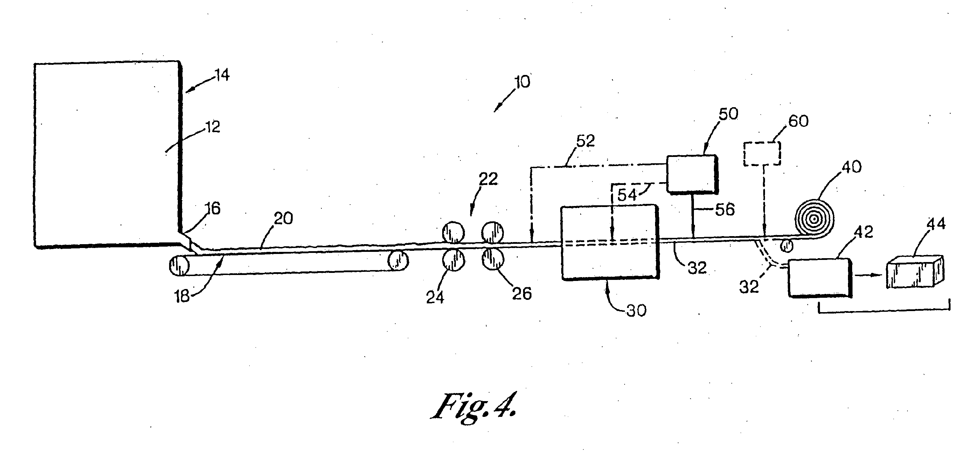

FIGURE 4 is a schematic illustration of a wet laid web manufacturing line illustrating the application of a saccharide to a wet laid web of cellulose fibers in accordance with the present invention; and

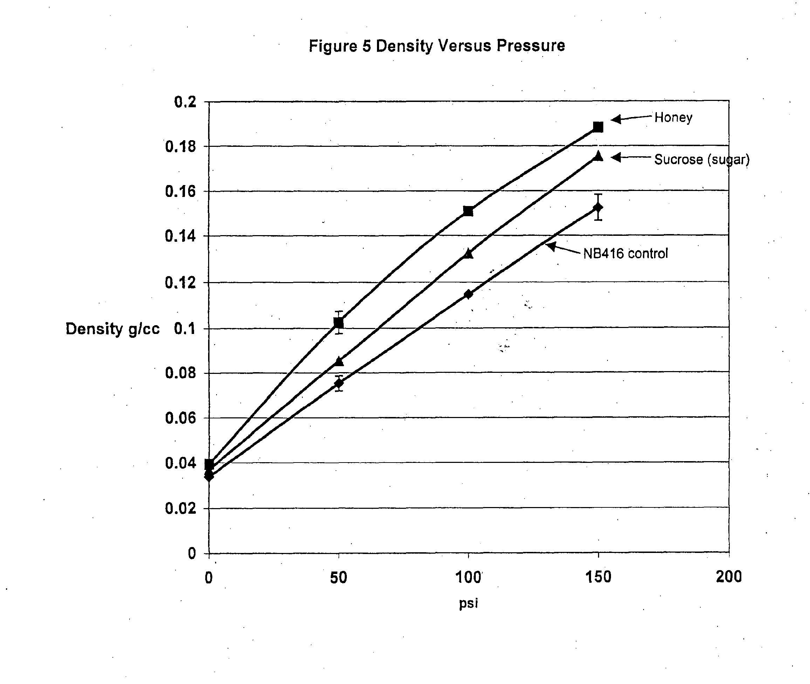

FIGURE 5 is a graph illustrating the difference in densification properties between fibers resulting from the fiberization of honey, sucrose and maltodextrine treated cellulose pulp sheets of the present invention and fibers resulting from the fiberization of cellulose pulp sheets that have not been treated in accordance with the present invention.

DETAILED DESCRIPTION OF THE PREFERRED EMBODIMENT

As used herein, the term saccharide refers to mono-, di-, oligo-, and polysaccharides.

Monosaccharides are carbohydrates that cannot be hydrolyzed into smaller, simpler carbohydrates. Examples of monosaccharides include glucose, fructose, glyceraldehyde, dihydroxyacetone, erythrose, threose, ribose, deoxyribose, galactose, and the like.

Disaccharides are carbohydrates that on a molar basis undergo hydrolysis to produce only two moles of a monosaccharide. Examples of disaccharides include maltose, sucrose, cellubiose, lactose, and the like.

Oligosaccharides are carbohydrates that on a molar basis undergo hydrolysis to produce 3 to 10 moles of a monosaccharide. Examples of oligosaccharides include those found in corn syrups and other mixtures of breakdown products from polysaccharides, and the like.

Polysaccharides are carbohydrates that on a molar basis undergo hydrolysis to produce more than ten moles of a monosaccharide. Examples of polysaccharides include starch, chitin, hemicelluloses such as galactomannan, other polysaccharides found in seaweed, and the like.

It should be understood that the term saccharide as used herein not only refers to individual saccharides such as glucose, fructose, or lactose, but also includes mixtures of monosaccharides, disaccharides, oligosaccharides, and/or polysaccharides.

Examples of saccharides that include a mixture of monosaccharides, disaccharides, oligosaccharides, or polysaccharides include corn syrup, high fructose corn syrup, and honey. Corn syrup is generally a mixture of dextrose (glucose), maltose, and maltodextrines and is available from numerous commercial sources. High fructose corn syrup generally includes fructose, dextrose, disaccharides, and other saccharides. Com syrup and high fructose corn syrup typically are available as aqueous solutions and have a solids content that ranges from 70 to 85 wt. %. An exemplary high fructose corn syrup is available from Archer-Daniels Midland Company under the trademark Corn Sweet® 42. It should be understood that other high fructose corn syrups that are available from Archer-Daniels Midland Company and other commercial sources are useful in the present invention.

Honey useful in accordance with the present invention contains fructose and glucose as the predominate carbohydrates, with maltos and sucrose present in small percentages. Honey is available from numerous commercial sources.

Exemplary cellulose pulp sheets useful in the present invention are derived from plant sources such as cotton, flax, bagasse, hemp, jute, rice, wheat, bamboo, corn, sisal, kenaf, peat moss, and the like. Preferred cellulose pulp sheets are wood pulp sheets such as those described in U.S. Patent No. 5,789,326. Generally, such wood pulp sheets can be produced by a chemical, thermomechanical, or chemithermomechanical process. Suitable wood pulp sheets may also be pretreated prior to the application of the saccharide in accordance with the present invention. Examples of suitable pretreatments include cross-linking the fibers, treating the fibers to effect their wettability, or bleaching the fibers. Additionally, other fibers, natural or synthetic, may be included in the pulp sheet. Examples of other fibers include silk, wool, linen, rayon, lyocell, polyethylene, polypropylene, polyester, polyamide, and the like.

As used herein, the term knot refers to a mass of individual fibers that have not been separated from each other. Fiberized masses of fibers having lower knot levels are preferred over fiberized masses that have higher knot levels because the presence of knots generally viewed as preventing the fibers from performing their functions of distribution of any superabsorbent polymer (if present), providing pad integrity, fluid distribution and liquid holding capacity. Excessive knots are also viewed as unsightly and detrimental to the visual acceptance of the finished product. Saccharide-treated cellulose pulp sheets of the present invention when fiberized under standard Kamas conditions described below, yield less than 20 wt. % knots as determined by the test described below in more detail. These fibers may be densified via the application of pressure in a press or nip-roll.

The solids content of the saccharide applied to the cellulose pulp sheet is less than about 65 wt. %. When the saccharide has a solids content less than about 65 wt. %, the cellulose pulp sheet treated with the saccharide when fiberized under standard Kamas conditions yields less than about 20 wt. % knots. When corn syrup, high fructose corn syrup, honey, or sucrose is used as the source of saccharide, it can be diluted with water in order to reduce the solids content to below about 65 wt. %. Preferably, no other agents are used to dilute the saccharide, such as organic solvents so that the cellulose pulp sheets of the present invention preferably consist only of cellulose fibers, saccharide, and water introduced to the cellulose fibers with the saccharide and water that is present as moisture in the cellulose pulp sheet.

With the particular saccharides corn syrup and high fructose corn syrup, which are typically available with a solids content on the order of 70% or greater, diluting the corn syrup or high fructose corn syrup to a solids content of less than about 65 wt. % surprisingly results in a cellulose pulp sheet that when fiberized under standard Kamas conditions yields less than 20 wt. % knots. Heat treating saccharide treated cellulose pulp sheets as described in the examples also results in treated sheets that can be fiberized under standard Kamas conditions yielding less than 20 wt. % knots. Furthermore, as illustrated in the examples, treated pulp sheets which are not fiberized for 1-2 months after saccharide treatment yield less than 20 wt. % knots.

In accordance with the present invention, the amount of saccharide added to the cellulose pulp sheet can vary across a wide range. On the upper end, the amount of saccharide added to the cellulose pulp sheet is generally limited to an amount that maintains the water content of the cellulose pulp sheet below about 20 wt. %. When the saccharide has a solid contents less than 65 wt. % as described above, amounts of saccharide up to about 65 wt. % based on the dry weight of the fiber can be added. Adding lesser amounts of saccharide to the cellulose pulp sheet will provide the improved densification and softness properties of the fluff produced from such saccharide treated cellulose pulp sheet.

In preferred embodiments of the present invention, the saccharide is added to the cellulose pulp sheet in an amount that results in a dry solids content of less than about 20 wt. % based on the weight of dry cellulose fiber in the treated cellulose pulp sheet and more preferably less than 10 wt. %. As noted above, such dry solids content can be achieved using a saccharide that has been diluted with water. The extent of the dilution of the saccharide, and therefore the ratio of water to saccharide solids, should be selected so that the desired degree of saccharide solids can be achieved without introducing so much water that the pulp sheet becomes difficult to fiberize due to the addition of excess water. It is well known that pulp which is too wet is difficult to fiberize. The dilution should not be so great that when the saccharide solution is added to the cellulose pulp sheet to achieve the desired saccharide content, the saccharide-treated cellulose pulp sheet yields more than 20 wt. % knots when fiberized under standard Kamas conditions. A water content of the cellulose pulp sheet after being treated with saccharide of about 10 wt. % based on the weight of the total product is exemplary. Lower water contents are contemplated with an upper limit of about 15-20 wt. % based on the need to avoid poor fiberization.

Sufficient amounts of saccharide should be added to the cellulose pulp so that when the cellulose pulp is fiberized, the knot yield is below 20 wt. % and the resulting fibers exhibit ease of densification properties and/or softness properties that are superior to the ease of densification and softness properties of the cellulose pulp without being treated with a saccharide. Improved ease of densification properties result when about 5.0 wt. % dry saccharide solids based on the dry cellulose fiber content of the pulp sheet. Lower amounts of dry saccharide solids in the treated cellulose sheets are within the present invention; for example, amounts down to about 0.5 wt. % based on the dry fiber content of the cellulose pulp are within the scope of the present invention.

The saccharides are applied to the cellulose pulp sheet in a number of different ways. The present invention is not limited to any particular application technique. Examples of suitable application techniques include spraying, rolling, dipping, and the like. The saccharide can be applied to one or both sides of the cellulose pulp sheet.

The procedure for determining knot yield of a saccharide-treated pulp sheet is described below. The cellulose pulp sheet is first subjected to the standard Kamas fiberization conditions described below at about 70°F and relative humidity of about 50%. Standard Kamas fiberization conditions are carried out in a Kamas Cell Mill® laboratory hammermill fiberization unit available from Kamas Industri AB of Sweden (typ - Kvarn H. 01 M-NR 7.102.2516). The hammermill is operated at the following conditions:

| Parameter | Setting |
|---|---|
| Breaker bar gap | 4 mm |
| Exit screen hole size | 19 mm |
| Rotor speed | 3,000 rpm |
| Pulp sheet mass feed rate | 2.75 to 2.8 grams per second |
| Pulp sheet width | 2 inches |
| Hammermill rotor diameter (tip-to-tip distance) | 12 inches |
| Number of hammers around circumference of rotor | 4 |

The fiberized pulp is collected and tested for knot yield in the following manner.

The knot content is determined using a sonic knot device used to classify fiberized pulp into fractions based on screen mesh size. The first collected fraction is the large knots and is defined as that material that is captured by a No. 5 mesh screen. The second fraction is the intermediate knots and is defined as the material captured by a No. 8 mesh screen. The third fraction is the smaller knots and is defined as the material captured by a No. 12 mesh screen. The fourth fraction is the accepts, or the singulated fibers, and is defined as that material that passes through the No. 5, No. 8, and No. 12 mesh screens, but is captured by a No. 60 mesh screen. The separation is accomplished by sound waves generated by a speaker that are imposed upon a pre-weighed sample of the fiberized saccharide-treated cellulose pulp placed on the first layer No. 5 mesh screen that is near the top of a separation column where the speaker sits at the very top. After a set period of time, each fraction from the No. 5, No. 8, and No. 12 screens is removed from the separation column and is added back to the No. 5 screen for a second pass through the sonic test. After the set period of time, each fraction from the No. 5, No. 8, and No. 12 screens is removed from the separation column and weighed to obtain the weight fraction of knots. For the purposes of the present invention, the predetermined time was 6 minutes.

Referring to FIGURE 3, a more detailed description of the apparatus used for the sonic knot test is provided.

The device consists of a loudspeaker 1a housed inside a polycarbonate cylinder 1b. The loudspeaker is a Radio Shack 12" diameter model (Cat. No. 40-1034, power handling capability = 75W RMS and 150W max., with a nominal impedance of 8 Ohms.). Under operation it is fed by an amplifier which results in the generation of a square wave input signal (of 26 volts peak-to-peak) set to result in a continuous ultra-low frequency tone at 13.0-13.6 Hz. Sound from the speaker passes through a loose sheet of plastic bag material 2 that is sealed against the walls of the polycarbonate cylinder to protect the speaker from the fluff fibers.

The polycarbonate cylinder is connected via two spacer discs 3 to a Tyler equivalent 5 mesh screen 4 (USA Standard testing sieve, ASTME - 11 specification, No. 5). Which in turn is connected to two more spacer discs 5 and 6, an 8 mesh screen 7, two more spacer discs 8 and 9, a 12 mesh screen 10, then a 60 mesh screen 11, and finally a 200 mesh screen 12. All screens and spacers are from the same manufacturer and are of equal 12" diameter and "nest" tightly into one another to create a unified stack. A good seal between the various screens and spacers is maintained with tape and "O-rings" to prevent air leaks. At the base of the stack the exit from the 200 mesh screen is sealed by a plastic bag 13 containing a hole 14 in its wall of ~1.25" diameter (this allows escape of air from the stack). The device is supported by resting on the rim of screen 12.

Air is supplied to the unit at three locations 15, 16, and 17 into spacers 3, 5, and 9, respectively, via small (~3-5 mm diameter) pipes fitted with fan type spray nozzles at their ends. The function of the air is to assist in gently separating the fiber mass as it is processed. Just before the start of each sonic cycle, about 1 to 3 ml of anti-static solution ("Heavy Duty Staticide" #2002 from VWR Company Cat. #58611-225) is pumped into tube 15 by a peristaltic pump at 19. When the airflow is turned on at the start of the sonic cycle the anti-static solution in tube 15 is ejected via a spray nozzle located on the end of tube 15 as a fine mist spray over the pulp 18 which is lying on the 5 mesh screen below. The function of the anti-static is to prevent static clumping/clinging together of the fibers.

Air flow rate is targeted to be 5.4 to 5.6 SCFM at location 15; 4.3 to 4.6 SCFM at location 16, and 2.5 to 2.8 SCFM at location 17. The air supply to the unit is at ~6-7% relative humidity (RH) at room temperature. The whole unit is contained in a sound proof box operated in a lab at 50% RH and 70-75F.

Prior to initiation of the sonic knot test, the samples of fiberized cellulose prepared from the pulp sheets treated with saccharide are conditioned at 50 percent relative humidity at 23°C, for a minimum of 4 hours. Five gram samples of the fiberized cellulose pulp are employed in 3 separate runs. As a further preparatory matter, a 5 gram sample of fiberized cellulose pulp that has not been treated with a saccharide is run through the different screens in order to distribute anti-static material throughout the different screens.

The incoming air pressure should be 20-25 psi at rest, and 20-24 psi when running. Pressure settings for the respective screens are as follows:

| The Screen | Air Flow (SCFM) |
|---|---|
| 5 mesh | 5.4 to 5.6 |
| 8 mesh | 4.3 to 4.6 |
| 12 mesh | 2.5 to 2.8 |

The knot determination procedure begins by running two 5 gram samples of a control cellulose pulp (Weyerhaeuser Company CF 416 from Columbus, MS) that has been fiberized under the standard Kamas conditions. The weight of the two runs for each screen is recorded and then the weights for both runs is added together and the percentage of knot yield is calculated as described below in more detail. The collected knots for the control samples are not rerun. When the control samples fall within the ranges of 7-8 wt. % knots proceed on to the test of the treated samples. Attaining this level of knots on the CF 416 sample ensures that the sonic unit, air flow, and other parameters, are operating properly. If the control samples do not fall within the established ranges for knots, adjust the equipment so that it does. Adjustments can be made to the air flows and voltage driving the square wave to the speaker to achieve operation within the desired range.

Once the sonic fractionator has been calibrated as described above, the test samples of the fiberized treated cellulose pulp sheet can be evaluated in the following manner. As generally described above, the overall procedure is to run three 5 gram samples of the fiberized cellulose pulp, collect the knots from all three runs, and then run the collected knots combined for a second pass.

The first 5 gram sample of fiberized pulp is placed on the top screen and distributed uniformly by hand without compacting. The fiberized pulp should be broken up into at least 30 to 40 small pieces. The speaker assembly is placed on top of the upper screen. The anti-static material delivers 1-3 ml of liquid over the sample. The fractionator is started and cycled for six minutes, after which it is stopped. After the cycle stops, the anti-static pump delivers 1 to 3 ml of anti-static material into the airstream, preparing the equipment for the next sample to be tested. After the cycle is stopped, the knots collected on the 5, 18, and 12 mesh screens are gathered by hand and weighed separately to the nearest 0.01 gram. The knots are saved from all screens for the final pass described below. Care should be taken not to bang the screens on the counter to remove knots. Cellulose pulp stuck on the bottom or around the top edges of a screen should be added into the next screen in sequence. The accepts collected on the 60 mesh screen should be gathered by hand and weighed to the nearest 0.01 gram and saved for reweighing after the final pass described below. Fines collected on the 200 mesh screen should be gathered gently first by brush to collect as much as possible into one area on the edge of the screen. Once gathered, the screen can be turned upside down and tapped on a counter gently so that the fines fall out. The fines should be gathered with fingers or a plastic scraper and weighed to the nearest 0.1 gram and saved for reweighing after the final pass described below. The inside surface of the speaker assembly that fits on top of the 5 mesh screen should be checked visually or by feel for collection of pulp. Any pulp collected there should be removed after the end of each cycle and saved for later. At the end of the three 5.0 gram runs, the pulp collected from the speaker should be combined with the collected knots to be rerun.

The foregoing is then repeated with each of the addition two 5.0 gram samples. All the knots collected from the 5, 8, and 12 mesh screens from each of the three runs is then combined and placed into the top 5 mesh screen and distributed evenly. Any pulp collected from the inside of the speaker assembly as described above should be added to the knots on the top screen. The collected pulp should be broken up into small pieces.

The fractionator should then be run for one more six minute cycle. When the cycle stops, the knots collected on the 5, 8, and 12 mesh screens should be gathered by hand and weighed separately to the nearest 0.01 gram. The accepts on the 60 mesh screen should be collected after this final run and combined with the saved accepts from the first runs described above. The combined saved accepts should be weighed to the nearest 0.01 grams.

The fines collected on the 200 mesh screen during this final pass should be combined with the saved fines from the first passes described above. The combined fines should then be weighed to the nearest 0.01 gram.

Again, the inside surface of the speaker assembly should be checked for any miscellaneous pulp that has collected there. This collected pulp should be weighed to the nearest 0.01 gram and recorded as Miscellaneous Weight.

To determine the percent knot yield by the pulp sample, an Adjusted Starting Weight for the pulp should be determined by subtracting the Miscellaneous Weight from the Total Starting Pulp Weight:

$$\text{Adjusted Starting Weight} = \text{Total Starting Pulp Weight} - \text{Miscellaneous Weight} \qquad (1)$$

To determine the percent knot yield, divide the weight of the knots after the final run by the Adjusted Starting Weight and multiply by 100 to provide a percentage using the following formula:

$$(\text{Final Recorded Weight of Knots} \div \text{Adjusted Starting Weight}) \times 100 = \% \text{ Knot Yield} \qquad (2)$$

The foregoing calculation is done for each of the knot weights for their respective 5, 8, and 12 mesh screens. The total knot yield is then determined by adding the percentages of the 5, 8, and 12 mesh screens together.

Cellulose pulp sheets treated with a saccharide in accordance with the present invention when fiberized under standard Kamas fiberization conditions yields less than 20 wt. % knots using the test procedure described above. The fiberized pulp of the present invention exhibits densification and softness properties that are improved compared to fibers produced from the same cellulose pulp sheets that have not been treated in accordance with the present invention. The preparation of treated cellulose pulp sheets of the present invention and the sheet densification and softness properties of the fiberized pulp sheets are described in more detail in the examples that follow.

**Example 1**

**Preparation of High Fructose Corn Syrup Treated Cellulose Pulp**

Southern Pine fluff in sheet form available from Weyerhaeuser Company under the designation NB 416 from New Bern, North Carolina with a starting moisture content of 6 % by weight (based on total sheet weight) was coated in a Black Brothers gravure-type roll coater with a solution of corn syrup. The gravure coater results in the application of a uniform coating of the corn syrup solution over one entire surface of the pulp sheet from where it is rapidly soaked up by the sheet. The corn syrup was a high fructose corn syrup available from Archer-Daniels Midland Company of Decatur, Illinois under the trademark CORN SWEET® 42. The com syrup had a solids content of 71% with the balance being water. This corn syrup was diluted with water to a solid content of 54.5 wt. % based on total solution weight before its application to the wood pulp sheet. This 54.5 wt. % solution was applied to the wood pulp sheet at a rate of 15.5 parts solution to 100 parts of pulp sheet, resulting in a loading of active on a (dry basis) corn syrup solids of 9 wt. % based on the dry fiber content of the pulp sheet (equivalent to 7.3 wt. % corn syrup solids based on the total final product weight). The final total moisture content of the wood pulp cellulose sheet treated with corn syrup is 11.3 wt. % based on the total final product weight.

The treated sheet was stored in a plastic zippered bag for 24 hours at room temperature to allow the added moisture to migrate and reach equilibrium within the whole sheet. The sheet was then fiberized in a laboratory Kamas Mill Hammermill operating under the standard Kamas fiberization conditions described above. Knot content was measured using the sonic knot device described above and resulted in a knot yield of 19% by weight.

**Example 2**

**Preparation of High Fructose Corn Syrup Treated Cellulose Pulp Sheet Using Diluted Corn Syrup**

The procedure in Example 1 was reproduced except that the CORN SWEET® 42 corn syrup was diluted to a solids content of 52.2 wt. % based on total solution weight using water. 10.58 parts of the corn syrup solution were added to 100 parts of the NB 416 wood pulp cellulose sheet. This resulted in a loading of active (on a dry basis) corn syrup solids of 5.88 wt. % based on the dry fiber content (or an equivalent 5.0 wt. % corn syrup solid based on the total final product weight). The final total moisture content of the wood pulp sheet treated with corn syrup was 10 wt. % based on the total final product weight. Knot yield after fiberization was 16 % for the above sample.

**Example 3**

**Preparation of High Fructose Corn Syrup Treated Cellulose Pulp Sheet Treated With Heat**

An additional sample of the corn syrup treated wood pulp of Example 1 was further evaluated on a Fitz Hammermill feeding an M&J continuous airlaid pad forming device. A portion of the corn syrup treated pulp sheet of Example 1 was first run as-is on the Fitz Hammermill. The resultant fiberized pulp was collected. The remaining portions of the treated pulp sheet were sealed inside plastic bags double layered to prevent moisture loss and heated for 24 hours at 150°F in a laboratory oven. After heat treatment, the pulp sheet was allowed to cool back to room temperature while still in the plastic bags and was then fiberized on the Fitz Hammermill under the same conditions used for the non-heat-treated pulp. Both heated and non-heated pulps were tested for knot content by the sonic method described above. The knot content of the two samples was similar; however, the physical appearance of the pulp which received the heat treatment exhibited a marked lack of fiber clumping as compared to the unheated sample. The absence of fiber clumping for the heated sample is evidence of further improvement of fiberization quality (over that which is indicated by sonic knots alone). Absence of fiber clumps indicates the positive effect of heating during the period where the corn syrup is soaking into and distributing itself amongst the fibers of the sheet.

**Example 4**

**Preparation of High Fructose Corn Syrup Treated Cellulose Pulp Sheet After Storing at Room Temperature**

An NB 416 wood pulp sheet is treated with high fructose corn syrup in accordance with Example 1 described above. The treated sheet is stored at room temperature in zippered plastic bags to prevent moisture loss for a period of two months. After the storage time, the fiberization characteristics are retested by Kamas fiberization using the standard Kamas fiberization conditions and the knot content is evaluated using the sonic method. Knot content of the treated sheet was 12 wt. %, a significant drop compared to the knot content of the sheet tested in Example 1. The foregoing suggests that prolonged storage at room temperature of a wood pulp cellulose sheet treated with the corn syrup of Example 1 can effect the fiberization properties of the sample.

**Example 5**

**Densification and Softness Properties of Fibers Produced From High Fructose Corn Syrup Treated Cellulose Pulp Sheets**

High fructose corn syrup treated wood pulp sheets of NB 416 were prepared according to Example 2. The treated sheets were fiberized in a laboratory hammermill different from the Kamas Cell Mill® described above, but one which closer resembles the set-up of hammermills used in commercial fiberization operations. The fiberized pulp was fed to a commercial airlaid rotating circular pocket former of the type used on commercial diaper manufacturing lines.
The above fiberized com syrup treated pulp and untreated pulp were airlaid into pads of about 400 grams per square meter basis weight, measuring about 12 inches long and five inches wide. The pads were densified in a hydraulic flat press under loads of either 0 psi, 50 psi, 100 psi, and 150 psi. The pressure was only held momentarily and then released. Different pads were used for each of the successively higher loads. Caliper (thickness) of the pads was determined using a caliper gage with a wide "foot" design to apply only moderate pressure to the pad (i.e., it does not materially densify the pad in the act of determining caliper). The densities of the pads were calculated from the caliper and basis weight measurements.
Results of the density measurements versus applied pressure are shown in FIGURE 1 and show that the high fructose corn syrup treated pulp attains a higher density for a given pressure compared to the untreated pulp.
The softness of the airlaid pads was determined by measuring a cantilever "bend length" for the pad as the long axis of the pad is extended out over a 45° inclined plane. The distance between the edge over which the pad is pushed and the outermost tip of the pad where it touches down on the 45° inclined plane is recorded in centimeters as the "bend length," and provides a measure of relative softness. A long bend length implies a stiff (or less soft) pad. Results of the softness test (across the range of applied pressures) are shown in FIGURE 2 and illustrate that the high fructose corn syrup treated wood pulp fibers has softness properties superior to those of the untreated wood pulp cellulose fibers.

**Example 6**

**Preparation of Honey and Sucrose Treated Cellulose Pulp Sheets**

Pieces of southern pine pulp in sheet form available from Weyerhaeuser Company under the designation NB 416 from New Bern, North Carolina, having a starting moisture content of 6 wt. % based on the total sheet weight, were prepared measuring about 4 inches wide by 20 inches long. One hundred parts of the cellulose pulp sheet was coated with one of the following solutions using a laboratory syringe. The saccharide containing solution was applied on to one side of the cellulose pulp sheet. The first solution was an aqueous solution containing 44 wt. % solids of common table honey. The second solution was an aqueous solution containing 50 wt. % solids of white table sugar, i. e., sucrose. The honey containing solution was applied in an amount of 11.8 parts and the sucrose containing solution was applied to a separate sample of cellulose pulp sheet in the amount of 10.4 parts.

The treated sheets were placed in a sealed plastic bag for 24 hours and fiberized under the standard Kamas fiberization conditions. Knots were determined as described above and resulted in yields of 17 wt. % for the cellulose pulp sheet treated with the honey solution, and 15 wt. % for the cellulose pulp sheet treated with the sucrose solution.

Ease of densification for each of the two samples described above was determined by first preparing freshly fluffed material in a laboratory Waring blender, running for about 30-60 seconds on high speed, using approximately 1.5 grams of the treated pulp sheet per sample. The resulting 1.5 grams of fluff was formed into circular pads measuring about 7 cm in diameter using a laboratory airlaid pad former. The initial weight and caliper of the pads were determined and then each pad was subjected to momentary pressure in a flat press (laboratory Carver press) at each of three, successively higher pressures of 50 psi, 100 psi, and 150 psi. After pressing at each pressure, the caliper was redetermined using a caliper gauge that applies a modest pressure so that the pad is not materially compressed and these readings used to calculate density. The results are illustrated in FIGURE 5 and illustrate the densification properties of fibers prepared from cellulose pulp sheets treated with a saccharide in accordance with the present invention versus fibers produced by fiberizing the NB 416 pulp sheet described above.

The foregoing examples describe the present invention in the context of a particular saccharide, namely, high fructose corn syrup. It should be understood that the present invention is not necessarily limited to high fructose corn syrup and that other saccharides, such as those of Example 6, are considered to be within the scope of the present invention.

FIGURE 4 illustrates a wet laid sheet manufacturing line such as a pulp sheet manufacturing line 10 for manufacturing the treated cellulose pulp sheets of the present invention. In this manufacturing line, a pulp slurry 12 is delivered from a headbox 14 through a slice 16 and onto a Fourdrinier wire 18. The pulp slurry 12 typically includes wood pulp fibers and may also include synthetic or other non-cellulose fibers as part of the slurry. Water is drawn from the pulp deposited on wire 18 by a conventional vacuum system, not shown, leaving a deposited pulp sheet 20 which is carried through a dewatering station 22, illustrated in this case as two sets of calendar rolls 24, 26 each defining a respective nip through which the pulp sheet or mat 20 passes. From the dewatering station, the pulp sheet 20 enters a drying section 30. In a conventional pulp sheet manufacturing line, drying section 30 may include multiple canister dryers with the pulp mat 20 following a serpentine path around the respective canister dryers and emerging as a dried sheet or mat 32 from the outlet of the drying section 30. Other alternate drying mechanisms, alone or in addition to canister dryers, may be included in the drying stage 30. The dried pulp sheet 32 has a maximum moisture content pursuant to the manufacturer's specifications. Typically, the maximum moisture content is no more than 10% by weight of the fibers and most preferably no more than about 6% to 8% by weight. The dried sheet 32 is taken up on a roll 40 for transportation to a remote location, that is, one separate from the pulp sheet manufacturing line, such as at a user's plant for use in manufacturing products. The dried pulp sheets have a basis weight of about 200 g/m$^2$ to about 1000 g/m$^2$ or more and a density on the order of at least about 0.3 g/cm$^3$ and more preferably 0.5 g/cm$^3$. Dried cellulose pulp sheets having the foregoing basis weights and densities that are useful in the present invention are structurally distinct from lighter basis weight sheets of wet laid or airlaid wood pulp fibers such as crepe paper, tissue paper, paper towels, or other types of paper-like wet laid or airlaid webs of cellulose fibers. Alternatively, the saccharide can be applied in a manufacturing line that does not employ a Fourdrinier wire and produces cellulose pulp in non-sheet form, such as chunks or slabs, that can be collected in a baling apparatus 42 from which bales of the pulp 44 are obtained for transport to a remote location. The cellulose pulp in non-sheet form useful in the present invention has a density that is greater than about 0.3 g/m$^3$ and more preferably greater than about 0.5 g/m$^3$.

A saccharide of the type explained in detail below is applied to the pulp sheet from one or more saccharide applying devices, one of which is indicated at 50 in FIGURE 4. Any applying device may be used, such as streamers, sprayers, roll coaters, curtain coaters, immersion applicators, or the like. Sprayers are typically easier to utilize and incorporate into a pulp-sheet manufacturing line. As indicated by the arrows 52, 54, and 56, the fiber treatment composition may be applied at various locations or at multiple locations on the pulp sheet manufacturing line, such as ahead of the drying stage 30 (indicated by line 52), intermediate the drying stage 30 (as indicated by line 54), or downstream from the drying stage 30 (as indicated by the line 56). Application of the saccharide after some drying has taken place, for example at location 54, is preferable and more preferably at 56 after the drying stage. If the saccharide is applied at location 56 in an amount which would cause the moisture content of the sheet to exceed the desired maximum level, an additional drying stage (not shown) may be included in the pulp manufacturing line to bring the moisture content down to the desired level.

The rolls 40 or bales 44 of the treated wet laid web of fibers may be transported to a remote location for use by a user. These rolls or bales are then refiberized by a fiberizing device, such as a hammermill which may be used alone or in conjunction with other devices such as picker rolls or the like for breaking up the sheet 32 or bales 42 into individual fibers. Depending on the end use, the individualized fibers may be combined with particulate material, such as superabsorbent particles, and/or airlaid into a web and densified.

With this approach, the end user of the treated fibers may readily select particles to be combined with the fibers. The user has flexibility in air laying or otherwise processing the treated fibers of the present invention into a finished product.

While the preferred embodiment of the invention has been illustrated and described, it will be appreciated that various changes can be made therein without departing from the spirit and scope of the invention.

**Claims**

1.  A cellulose pulp sheet comprising:

    cellulose fibers; and
    a saccharide, wherein the pulp sheet when fiberized under standard Kamas conditions yields less than 20 wt. % knots.

2.  The sheet of Claim 1 having a density greater than 0.3 g/cm$^3$.

3.  The sheet of Claim 1 or 2, wherein the cellulose fibers are wood pulp fibers.

4.  The sheet of any one of the preceding Claims wherein the saccharide is a corn syrup.

5.  The sheet of Claim 4 wherein the corn syrup is a high fructose corn syrup.

6.  The sheet of Claim 4 or 5 wherein the corn syrup is applied at a solids content of less than 65 wt.%.

7.  The sheet of any one of Claims 4 to 7 wherein the corn syrup includes solids that are present in an amount less than 10 wt.% based on the weight of dry cellulose fiber in the cellulose pulp sheet.

8.  A sheet according to any one of the preceding Claims consisting essentially of:

    the cellulose fibers; and
    the saccharide.

9.  A method for producing a saccharide treated cellulose pulp sheet that when fiberized under standard Kamas conditions yields less than 20 wt.% knots and as defined in any one of the preceding Claims, said method comprising:

    providing a cellulose pulp sheet; and
    applying a saccharide to the cellulose pulp sheet.

10. A cellulose pulp in non-sheet form comprising:

    a mass of cellulose fibers, the mass having a density of at least 0.3 g/cm$^3$; and
    a saccharide, wherein the cellulose pulp in non-sheet form, when fiberized under standard Kamas conditions yields less than 20 wt.% knots.

11. An absorbent product comprising a fiberized cellulose pulp sheet of any one of Claims 1 to 8.

**12.** An absorbent product comprising a fiberized cellulose pulp of Claim 10.

EP 1 457 184 A1

Figure 1 Density Versus Pressure

Figure 2 Bend Length vs. Density

Figure 3.

*Fig. 4.*

Figure 5 Density Versus Pressure

EP 1 457 184 A1

**European Patent Office**

## EUROPEAN SEARCH REPORT

Application Number

EP 04 25 1198

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | US 3 903 889 A (TORR DECEASED DAVID) 9 September 1975 (1975-09-09) * column 3, line 33 - line 37 * * column 5, line 19 - line 31 * * column 5, line 47 - line 52 * * column 7, line 5 - line 16 * ----- | 1-5,8-12 | A61F13/15 A61L15/20 A61L15/22 A61L15/28 |
| X | WO 95/22655 A (WEYERHAEUSER CO) 24 August 1995 (1995-08-24) * page 16, line 21 - page 17, line 1 * * page 41, line 26 - line 29 * ----- | 1-3,8-12 | |
| X | US 2002/096291 A1 (YOUNG RICHARD H  ET AL) 25 July 2002 (2002-07-25) * page 6, paragraph 3 * * page 27, paragraph 3 * ----- | 1-3,8-12 | |
| X | WO 02/087644 A (ACORDIS SPECIALITY FIBRES LTD ; EDMONDS JAMES WILLIAM (GB)) 7 November 2002 (2002-11-07) * page 2, line 27 - line 31 * * page 3, line 28 - line 30 * * page 5, line 2 - line 8 * ----- | 1-3,8-12 | TECHNICAL FIELDS SEARCHED (Int.Cl.7) A61F A61L |
| X | US 6 284 943 B1 (HINES LETHA MARGIE  ET AL) 4 September 2001 (2001-09-04) * column 10, line 5 - line 13 * * column 14, line 47 - line 63 * * claim 10 * ----- | 1-3,8-12 | |

The present search report has been drawn up for all claims

| Place of search | Date of completion of the search | Examiner |
|---|---|---|
| Munich | 8 July 2004 | Staber, B |

EPO FORM 1503 03.82 (P04C01)

**ANNEX TO THE EUROPEAN SEARCH REPORT
ON EUROPEAN PATENT APPLICATION NO.**                    EP 04 25 1198

This annex lists the patent family members relating to the patent documents cited in  the above-mentioned European search report.
The members are as contained in the European Patent Office EDP file on
The European Patent Office is in no way liable for these particulars which are merely  given for the purpose of information.

08-07-2004

| Patent document cited in search report | | Publication date | Patent family member(s) | | Publication date |
|---|---|---|---|---|---|
| US 3903889 | A | 09-09-1975 | NONE | | |
| WO 9522655 | A | 24-08-1995 | US | 5547541 A | 20-08-1996 |
| | | | AT | 236295 T | 15-04-2003 |
| | | | AU | 1921395 A | 04-09-1995 |
| | | | CA | 2181476 A1 | 24-08-1995 |
| | | | CA | 2412892 A1 | 24-08-1995 |
| | | | DE | 69530186 D1 | 08-05-2003 |
| | | | DE | 69530186 T2 | 29-01-2004 |
| | | | EP | 0745160 A1 | 04-12-1996 |
| | | | ES | 2197916 T3 | 16-01-2004 |
| | | | JP | 9509226 T | 16-09-1997 |
| | | | WO | 9522655 A1 | 24-08-1995 |
| | | | US | 6340411 B1 | 22-01-2002 |
| | | | US | 5807364 A | 15-09-1998 |
| | | | US | 2002096291 A1 | 25-07-2002 |
| | | | US | 2002096292 A1 | 25-07-2002 |
| US 2002096291 | A1 | 25-07-2002 | US | 6340411 B1 | 22-01-2002 |
| | | | US | 5547541 A | 20-08-1996 |
| | | | US | 5672418 A | 30-09-1997 |
| | | | US | 5607759 A | 04-03-1997 |
| | | | US | 5693411 A | 02-12-1997 |
| | | | US | 5547745 A | 20-08-1996 |
| | | | US | 5641561 A | 24-06-1997 |
| | | | US | 5543215 A | 06-08-1996 |
| | | | US | 5538783 A | 23-07-1996 |
| | | | US | 5300192 A | 05-04-1994 |
| | | | US | 5352480 A | 04-10-1994 |
| | | | US | 5308896 A | 03-05-1994 |
| | | | US | 5589256 A | 31-12-1996 |
| | | | US | 2002096292 A1 | 25-07-2002 |
| | | | AT | 236295 T | 15-04-2003 |
| | | | AU | 1921395 A | 04-09-1995 |
| | | | CA | 2181476 A1 | 24-08-1995 |
| | | | CA | 2412892 A1 | 24-08-1995 |
| | | | DE | 69530186 D1 | 08-05-2003 |
| | | | DE | 69530186 T2 | 29-01-2004 |
| | | | EP | 0745160 A1 | 04-12-1996 |
| | | | ES | 2197916 T3 | 16-01-2004 |
| | | | JP | 9509226 T | 16-09-1997 |
| | | | WO | 9522655 A1 | 24-08-1995 |
| | | | US | 5807364 A | 15-09-1998 |
| | | | US | 2002164375 A1 | 07-11-2002 |
| | | | US | 6071549 A | 06-06-2000 |
| | | | US | 6395395 B1 | 28-05-2002 |

EPO FORM P0459

For more details about this annex : see Official Journal of the European Patent Office, No. 12/82

# EP 1 457 184 A1

## ANNEX TO THE EUROPEAN SEARCH REPORT
## ON EUROPEAN PATENT APPLICATION NO.

EP 04 25 1198

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2004

| Patent document cited in search report | | Publication date | Patent family member(s) | | Publication date |
|---|---|---|---|---|---|
| US 2002096291 | A1 | | US | 5998032 A | 07-12-1999 |
| | | | US | 5789326 A | 04-08-1998 |
| | | | US | 5611885 A | 18-03-1997 |
| | | | US | 2002025435 A1 | 28-02-2002 |
| | | | US | 2003201051 A1 | 30-10-2003 |
| | | | US | 6521339 B1 | 18-02-2003 |
| | | | US | 6596103 B1 | 22-07-2003 |
| | | | US | 6461553 B1 | 08-10-2002 |
| | | | US | 2001021453 A1 | 13-09-2001 |
| | | | AT | 225708 T | 15-10-2002 |
| | | | AT | 224808 T | 15-10-2002 |
| | | | AU | 5019893 A | 15-03-1994 |
| | | | AU | 5019993 A | 15-03-1994 |
| | | | BR | 9306920 A | 12-01-1999 |
| | | | BR | 9306921 A | 12-01-1999 |
| | | | CA | 2140263 A1 | 03-03-1994 |
| | | | CA | 2140264 A1 | 03-03-1994 |
| | | | DE | 69332336 D1 | 31-10-2002 |
| | | | DE | 69332336 T2 | 30-01-2003 |
| | | | DE | 69332380 D1 | 14-11-2002 |
| | | | DE | 69332380 T2 | 13-02-2003 |
| | | | EP | 1217120 A1 | 26-06-2002 |
| WO 02087644 | A | 07-11-2002 | WO | 02087644 A1 | 07-11-2002 |
| US 6284943 | B1 | 04-09-2001 | AT | 251437 T | 15-10-2003 |
| | | | AU | 731915 B2 | 05-04-2001 |
| | | | AU | 5525198 A | 03-07-1998 |
| | | | CA | 2274830 A1 | 18-06-1998 |
| | | | DE | 69725471 D1 | 13-11-2003 |
| | | | EP | 0966242 A1 | 29-12-1999 |
| | | | JP | 2000505688 T | 16-05-2000 |
| | | | JP | 3481633 B2 | 22-12-2003 |
| | | | WO | 9825564 A1 | 18-06-1998 |

EPO FORM P0459

For more details about this annex : see Official Journal of the European Patent Office, No. 12/82

19